# EUROPÄISCHE PATENTSCHRIFT

(11) **EP 3 801 124 B1**
(45) Veröffentlichungstag und Bekanntmachung des Hinweises auf die Patenterteilung: **31.12.2025**
(21) Anmeldenummer: 19729496.0
(22) Anmeldetag: 04.06.2019
(51) Int. Cl.: A46B 3/18, A46B 9/02, A46D 1/00, A61B 10/02, A61B 17/32

(54) **PROBEENTNAHMEANORDNUNG FÜR GYNÄKOLOGISCHE UNTERSUCHUNGEN**
SAMPLE REMOVAL ASSEMBLY FOR GYNECOLOGICAL EXAMINATIONS
ENSEMBLE DE PRÉLÈVEMENT D'ÉCHANTILLONS POUR DES EXAMENS GYNÉCOLOGIQUES

(30) Priorität: 04.06.2018 DE 102018208769
(43) Veröffentlichungstag der Anmeldung: 14.04.2021
(73) Patentinhaber: Mediscene AG, 4052 Basel (CH)
(72) Erfinder: RINDERKNECHT, Bernhard, 4052 Basel (CH)
(74) Vertreter: Pietruk, Claus Peter
(86) Internationale Anmeldenummer: PCT/EP2019/064534
(87) Internationale Veröffentlichungsnummer: WO 2019/234048

(56) Entgegenhaltungen:
- WO-A1-2016/104779
- KR-B1- 101 504 264
- US-A1- 2009 062 691
- US-A1- 2013 338 533

## Beschreibung

Die vorliegende Erfindung betrifft das oberbegrifflich Beanspruchte und bezieht sich somit auf das Gebiet der gynäkologischen Untersuchungen.

Bei vielen gynäkologischen Untersuchungen und Vorsorgeuntersuchungen ist es erforderlich, Abstriche im Bereich des Muttermundes (Cervix uteri) oder der Scheide (Vagina) durchzuführen und das so gewonnene Material labortechnisch zu untersuchen.

Eine Schlüsselrolle in der präventiven Medizin spielt die Prävention des Cervixkarzinoms, die bisher wirksamste primärpräventive Vorsorgemassnahme überhaupt, bei der durch Zellentnahme die mikroskopisch gut erkennbaren Krebsvorstufen (Dysplasien) des Gebärmutterhalses (Cervix uteri) identifiziert werden können. Dazu werden seit den 1950er Jahren Tests durchgeführt, die man aufgrund der 1928 beschriebenen Erstbeobachtung von Prof. George Papanicolaou Pap-Tests nennt. Bei diesem Zellabstrich werden Zellen vom Gebärmutterhals (Cervix uteri) der Frau gewonnen, gefärbt und mikroskopisch untersucht. Die Zellen werden dabei vom Untersucher auf einen Glasobjektträger ausgestrichen und mit einer alkoholischen Lösung fixiert. Das Material (Zellen und mitentnommene mucopolysachharidhaltige Schleimsubstanz) kann auch in einen z.B. mit Ethanol, Isopropanol oder Methanol flüssigkeitsgefüllten kleinen Behälter überführt werden, der das Zellmaterial fixiert, eine Zelldegeneration verhindert und Zellmaterial für die weitere Aufarbeitung konserviert. Solche Proben können nicht nur cytologisch-zellmikroskopisch, sondern auch molekularbiologisch untersucht werden, z.B. für HPV-Human Papilloma Virus-Nachweise, Biomarker, wie z.B. p16 und Ki-67. Die unterschiedlichen Lösungsmittel müssen dabei so ausgesucht werden, dass das gewonnene Material nicht verklumpt und dass die nachfolgende Analyse ordnungsgemäß ermöglicht ist.

Die entsprechenden Untersuchungen werden heute standardmäßig in großer Zahl durchgeführt.

Um das korrekte Material in möglichst grosser Menge für die nachgestellten Untersuchungen zu gewinnen, muss der damit verbundene sogenannte präanalytische Methodenfehler minimiert werden, was einer Erhöhung der Methoden-Sensitivität entspricht. Die beiden dabei bestimmenden Faktoren sind erstens Material von der richtigen Stelle und zweitens Materialmenge.

Die Anatomie des Entnahmeortes, der Gebärmutterhals (Cervix uteri) unterliegt einer für den menschlichen Körper fast einzigartigen makroskopischen und mikroskopischen, lebenslangen Umgestaltung. Durch die biologische Fortpflanzungskraft erfährt die Cervix uteri im Leben einer Frau vor allem durch den Einfluss der beiden weiblichen Haupthormongruppen, den Östrogenen und den Gestagenen, sehr starke Umwandlungen. Mikroskopisch (feingeweblich) ist die Cervix uteri von zwei Oberflächenzellschichten (Epithelien) bedeckt und zwar dem dickeren mehrschichtigen Platten- und dem einschichtigen Zylinderepithel. Die Zone, wo sich die beiden Epithelien treffen und über einen sogenannten Metaplasieprozess teilweise in einander umgewandelt werden, nennt man Transformationszone, Umwandlungszone, Übergangszone oder Junktionszone. Die in der Cervixkarzinomprophylaxe relevante Region ist diese Zone. In der Regel findet dort der Kanzerisierungsprozess, d.h. die Entstehung der Krebsvorstufen (Dysplasien) statt. Ausserdem ist vor allem das kranial an die Umwandlungszone anschliessende einreihige Zylinderepithel durch seine "dünnhäutige" Vulnerabilität bevorzugter Aufenthalts- und Eintrittsort für gewisse Infektkeime. Die Umwandlungszone "wandert" vom Inneren der Cervix uteri (Endocervix) beim Mädchen durch den Einfluss der Östrogene in der Pubertät und Adoleszenz auf die Ektocervix (Ektopie, Ektropion), in der Schwangerschaft oft noch weiter hinaus (Schwangerschaftsektopie), um sich dann nach der Menopause wieder in die Endocervix zurück zu verlagern. Ausserdem erfährt der makroskopische Bau und die Form der Cervix durch Geburten, Operationen, wie Gebärmutterhalsumschneidungen und den oestrogenmangelbedingt akzelerierten Atrophieprozess (geweblicher Alterungsprozess) teils starke Formveränderungen.

Dies kann zu Problemen bei nachfolgenden Untersuchungen der gewonnenen Proben führen. Wird die Probe nämlich an einer falschen Stelle, insbesondere zu weit innen, gewonnen, so besteht die Gefahr, dass inkorrekte Laborergebnisse erhalten werden. Wünschenswert ist dabei ein für alle anatomischen Gegebenheiten, d.h. z.B. altersunabhängig einsetzbares Instrument.

Ungeachtet dessen soll das Abstrichinstrument möglichst für alle Situationen eine fachgerechte Materialentnahme erlauben und möglichst viel Material "sampeln". Dabei sollte das Instrument durch seinen Bau zugleich eine standardisierte "fool-proofed" Materialentnahme ermöglichen, die prinzipiell auch paramedizinischem Personal ohne anatomische Grundkenntnisse eine sichere und valide Probennahme ermöglichen kann.

Die Durchführung eines Abstriches wird in der weit überwiegenden Zahl der Fälle als unangenehm, gelegentlich sogar als schmerzhaft empfunden. Daher ist es sinnvoll, die Materialgewinnung so durchzuführen, dass der eigentliche Eingriff an der Patientin kurz ist und Wiederholungen entbehrlich sind. Zudem sollten, wie erwähnt, auch weniger erfahrene Ärzte und paramedizinisches Personal nach Einführung in die Lage versetzt werden, das erforderliche Probenmaterial problemfrei und sicher zu gewinnen.

Die Probennahme als Massenuntersuchung muss zudem mit preiswerten Instrumenten erfolgen, bei denen es sich heute ausschliesslich und auch vorliegend um Einweg-Instrumente handelt.

Der PAP-Test von Papanicolaou wurde ursprünglich mit Vaginalzellen durchgeführt, die mit einer Pipette gewonnen wurden. In der Folge haben der sog. Ayre Spatel und dann die Entwicklung des sog. Cytobrush durch Nils Stormby zu einer deutlichen Sensitivitätssteigerung geführt. Die Anwendung dieser Bürste erfolgt typisch mit einem Spekulum und unter Sichtkontrolle. Gleichwohl kann es passieren, dass die Bürste nicht bis an die richtige Stelle bewegt wird, wobei sie von unerfahrenen Ärzten insbesondere zu weit eingeschoben wird. Zudem ist für die Gewinnung von Proben zusätzlich die Verwendung eines Spatels geboten, um Material von allen anatomisch relevanten Bereichen zu gewinnen.

Aus der US PS 3,881,464 ist bereits eine Probennahme-Vorrichtung bekannt, bei welcher eine Probe aus dem Gebärmutterhals gewonnen wird, die für sowohl cytologische als auch histologische Untersuchungen geeignet sein soll, wozu eine Gebärmutterhalsprobe mit Gewebe und zellulärem Material durch In-Kontakt-Bringen der Cervix mit einer allgemein konischen Bürste gewonnen wird. Die Borsten dieser Bürste sollen in dem später verwendeten Lösungsmittel löslich sein, aber unlöslich in herkömmlichen Fixiermitteln für Zellen. Die allgemein konische Form soll dergestalt erreicht werden, dass die Borsten allgemein radial von einem Schaft wegführen. Am kaudalen, also patientenaußenseitigen Ende beträgt dieser Durchmesser etwa 2,5 cm. Der Durchmesser verringert sich über eine Strecke von ca. 2 cm auf 0,5 cm mit einer etwa linearen Abnahme, und es schließt sich kranial (zum Patienteninneren hin) ein ca. 1 cm langes Stück mit ungefähr gleichbleibendem Durchmesser von 0,5 cm an.

Die US 2009/0240164 A1 zeigt eine Bürste für gynäkologische Untersuchungen, die im Uterus verwendet werden soll. Einleitend wird erwähnt, dass verschiedene Bürsten verschiedenen Begrenzungen unterworfen sind, etwa dass nicht genügend Zellen gesammelt werden, dass ihre Benutzung als unbequem empfunden wird, dass die Einschubtiefe fraglich sein kann usw.

Die DE 699 28 281 T2 zeigt eine Abstrichbürste. Einleitend erwähnt wird der Abstrich im Gebärmutterhalskanal, und es wird auf die "Cytobrush" der Firma Medscand hingewiesen. Nachteilig soll daran sein, dass dort Nylonhaare mit stumpfem Werkzeug auf die richtige Länge geschnitten würden, was spitze Haarenden verursachen soll, und dass der Draht steif sei. Die DE 699 28 128 T2 schlägt vor, dass ein Bürstenkopf im Spritzgussverfahren hergestellt wird; die Haardichte soll zwischen 200-400 Haaren pro Quadratzentimeter betragen.

Verwiesen wird im Übrigen weiter darauf, dass gynäkologische Probeentnahmeinstrumente auch bekannt sind aus der DE 83 20 917 U1, der US 2009 / 0 062 691 A1, der US 2014/0 083 213 A1 sowie der WO 2016/104 779 A1 sowie der KR 101 504 264 B1. Dabei zeigt insbesondere die KR 101 504 264 B1 eine abnehmbare medizinische Bürste, die den Benutzer in die Lage versetzt, die Bürste zur Diagnose von Gebärmutterhalskrebsvorstufen einzuführen, um muköses Material zu gewinnen, wobei die Bürste von einem Griff separierbar und in einem Behälter platzierbar ist. Die Bürste ist kaudal mit einem aufkelchenden Bereich von Fasern gebildet, deren Enden nach kranial und dabei leicht nach außen zeigen. An diesen aufkelchenden Bereich schliesst sich kranial ein Bereich mit Borsten an, die sich radial nach außen erstrecken, eine geringere Länge als jene des aufkelchenden Bereiches aufweisen und dabei allgemein konisch nach kranial zulaufen. Die Bürste ist mittels eines Paares verdrillter Drähte gebildet, zwischen welchen die Borsten gehalten sind. Die Borsten des proximalen Bereiches ergeben aufgrund ihrer recht großen Länge den Eindruck einer durchgehenden Mantelfläche, was durch eine Aufspreizung der Borsten weg von der durch die verdrillten Drähte gebildeten Bürstenachse bewirkt ist.

Eine vergleichbare Bürste wurde vom Erfinder der vorliegenden Anmeldung ebenfalls über lange Zeit verwendet. Dabei zeigte sich allerdings, dass die eingangs genannten Probleme wie einfacher Eingriff, sichere Gewinnung bei unterschiedlichsten anatomischen Gegebenheiten und Verwendbarkeit mit einer Vielzahl unterschiedlicher labortechnischer Verfahren ohne das Erfordernis, für unterschiedliche Verfahren unterschiedliche Probeentnahmeanordnungen zu verwenden, noch nicht in einer unter allen Bedingungen vollständig zufriedenstellenden Weise gelöst werden.

Es ist daher wünschenswert, eine verbesserte Probeentnahmeanordnung für gynäkologische Untersuchungen der Vagina bzw. der Zervix, insbesondere gynäkologische VorsorgeUntersuchungen bereitzustellen, welche zumindest einige der vorgenannten Probleme zumindest partiell löst und die gleichwohl eine preiswerte Herstellung erlaubt.

Die Aufgabe der vorliegenden Erfindung besteht darin, Neues für die gewerbliche Anwendung bereitzustellen. Die Lösung dieser Aufgabe wird in unabhängiger Form beansprucht. Bevorzugte Ausführungsformen finden sich in den Unteransprüchen.

Die Erfindung schlägt somit unter anderem vor, dass bei einem gynäkologischen Probeentnahmeinstrument für Vaginal- bzw. Zervixuntersuchungen, das kranial eine Vielzahl von Borsten aufweist, die an einer sich in Axialrichtung erstreckenden Drahtanordnung fixiert sind, vorgesehen ist, dass die Borsten aus wenigstens 600 Kunststofffäden mit einem Durchmesser zwischen 350 µm und 900 µm und einer axialen Liniendichte von wenigstens 600 Fäden auf maximal 3 cm Axialerstreckung gebildet sind.

Ein erster Grundgedanke der vorliegenden Erfindung ist somit in der Erkenntnis zu sehen, dass eine sehr große Anzahl von Probeentnahmefäden hinreichender Dichte Vorteile sowohl bei Verwendung für Abstriche wie auch zur Überführung entnommenen Materials in eine Flüssigkeit bieten. Dies war nicht ohne weiteres zu erwarten. Obwohl die vergleichsweise dünnen Borsten jeweils nur wenig Material aufnehmen werden und auch insgesamt, ungeachtet der Vielzahl Kunststoff-Borsten, nur vergleichsweise wenig Material gewonnen wird, ergibt sich doch ein hervorragend geeignetes Probeentnahmeinstrument, da die bei der Probeentnahme gewonnenen Materialien sich sehr gut, insgesamt sogar wesentlich besser als im Stand der Technik, vom Probeentnahme-Instrument entfernen lassen.

Es kann dabei vorteilhaft sein, wenn die Borstendichte nicht übermäßig hoch wird, also Borsten weder zu dünn sind noch zu eng beieinander stehen. Sind die Fäden nämlich zu dünn, so wird von den entsprechenden Borsten bei Einzelstand zu wenig Material gesammelt. Liegen sehr dünne Borsten hingegen eng beieinander, wirken sich Kapillareffekte in den Borstenzwischenräumen zu stark aus. Dies kann dazu führen, dass anderes Material als ohne Kapillareffekte gesammelt wird, weil sich zum einen die kapillar aneinander haftenden Borsten wechselseitig etwas versteifen und weil zum anderen besonders feines Material im Untersuchungslabor eher an den Borsten haften bleibt, also ein anderer Anteil der gesammelten Zellen untersucht wird. Daher ist es bevorzugt, wenn die Borstendichte begrenzt wird. Liegt die Axialerstreckung des borstentragenden Bereiches bei z. B. 3cm, wird daher bevorzugt, wenn die Gesamtfadenzahl unter 1800 bleibt, bevorzugt unter 1500 und insbesondere bevorzugt unter 1200. Aus diesen Angaben sind für andere Axialerstreckungen Gesamtfadenzahlen einsichtigerweise herleitbar. Es wird aus der obigen Erklärung einsichtig sein, dass geringfügig dickere Fäden dem Kapillareffekt entgegenwirken und dass demgemäß die maximal sinnvolle Borstendichte dickenabhängig sein kann. Zudem wird einsichtig sein, dass die sinnvolle Gesamtfadenzahl von der Axiallänge abhängen wird, über welche hinweg Borsten vorgesehen sind. Aus den obigen Erläuterungen wird einzuschätzen sein, dass die Dichte nicht zu hoch sein soll.

Bei der Anfertigung von Präparaten für die Mikroskopie, die eine dünne Auftragung des Materials (Monolayer) auf einem Objektträger erfordert, ist es von Vorteil, dass an den dünnen Borsten jeweils nur wenig Material vorhanden ist, weil dies die Erzeugung dünner Ausstriche für die Mikroskopie erleichtert. Bei der Überführung des Materials in eine Flüssigkeit ist zu beachten, dass bei der gynäkologischen Untersuchung im Regelfall nicht nur die tatsächlich zu untersuchenden, gewünschten Zellen entnommen werden, sondern diese Zellen in einer Matrix anderen Materials wie Schleim oder dergl. eingebettet sein werden. Für labortechnische Untersuchungen ist dann erforderlich, die Zellen aus diesem Material zu entfernen. Müssen für einen späteren Untersuchungsprozess Lösungsmittel verwendet werden, in denen das Matrixmaterial, mit welchem die Zellen entnommen werden, verklumpt, etwa durch Koagulation oder dergleichen, ist die Gewinnung der Zellen äußerst schwierig.

Dass die Erfindung vorteilhaft ist, wird zumindest nach derzeitigem Kenntnisstand darauf zurückgeführt, dass nicht nur einige wenige große, sehr kompakte Klumpen gebildet werden, sondern allenfalls sehr viele kleine Klümpchen, in denen Zellen usw. entweder zumindest nicht so stark fixiert werden, wodurch die oberflächlich vorhandenen Zellen leicht abgetrennt werden können, oder aber sogar aufgrund der geringen Klumpen-Größe ohnehin ein geringerer Anteil an Zellen in deren Innerem vollständig eingeschlossen ist. Gleichzeitig erlaubt die Ausgestaltung der Bürste die Gewinnung von den bevorzugten Stellen.

Damit ist die Gesamtgewinnung von Untersuchungsmaterial mit dem gynäkologischen Probeentnahmeinstrument wesentlich verbessert. Zudem besteht durch die Verwendung geeignet dünner Fäden im Gegensatz zu harten Spateln, Instrumenten mit groben Borsten oder Schabern eine deutlich verringerte Neigung, durch die Probeentnahme bedingte Schmerzen oder Reizungen zu verursachen. Anders als Holz- und/oder Kunststoffspatel, Schaumstoffinstrumente oder Polyethyleninstrumente mit wenigen, sehr breiten "Zähnchen" sind die erfindungsgemäßen Instrumente zudem bautechnisch geeignet, in die histologisch ausgeformten tiefen Cervixkrypten einzugehen und das dort in der "Tiefe" gelegene zylinderepitheliale Zellmaterial zu gewinnen. Zudem sind die Borsten aufgrund ihres geringen Durchmessers noch so beweglich, dass durch die Probennahme bedingte Blutungen am Entnahmeort oder auf dem Weg dorthin bei Einführung des Probeentnahmeinstrumentes weitgehend, typisch sogar vollständig vermeidbar sind. Die Anwendung des Probeentnahmeinstrumentes ist demnach wesentlich angenehmer für eine Patientin.

Auch für den Arzt, der das gynäkologische Probeentnahmeinstrument einsetzen muss, ist damit bereits die Anwendung wesentlich einfacher, weil er weniger negative Rückmeldungen von den Patientinnen erhält. Dies erleichtert auch die Gewöhnung im Umgang mit dem erfindungsgemäßen gynäkologischen Probeentnahmeinstrument, zumal es Proben für eine Vielzahl unterschiedlicher Untersuchungen zu entnehmen geeignet ist und somit auch anfänglich unerfahrene Ärzte schneller an Erfahrung gewinnen.

Dies gilt selbst dort, wo für Vaginal- bzw. Zervixbürsten jeweils unterschiedliche Bürstenformen verwendet werden sollen, aber beide, also sowohl Vaginalbürste wie auch Zervixbürste zum Sammeln von Material für jeweils unterschiedliche nachgeordnete Untersuchungsverfahren verwendet werden sollen.

Es ist möglich und vorteilhaft, wenn bei einem gynäkologischen Probeentnahmeinstrument weiter und/oder zusätzlich vorgesehen ist, dass die Kunststofffäden aus Polyamid hergestellt sind. Polyamidfäden sind physiologisch gut verträglich und preiswert. Sie sind in einer Vielzahl unterschiedlicher Durchmesser verfügbar, sind preiswert und bieten bei den genannten erfindungsgemäßen Stärken eine Flexibilität, die für die Zellgewinnung an den erforderlichen Stellen ideal ist.

Es ist möglich, die Kunststofffäden mechanisch abzutrennen. Dabei ist möglich und vorteilhaft, wenn bei einem gynäkologischen Probeentnahmeinstrument insbesondere vorgesehen ist, dass die Kunststofffäden abgeschnitten bzw. abgequetscht oder abgeschert werden.

Es ist insbesondere möglich, dass das Ablängen auf die für die jeweilige Position innerhalb der Gesamtbürste erforderliche Länge schon vor der Fixierung im Borstenkörper erfolgt, was Verschnitt reduziert. Alternativ kann ein Beschnitt dergestalt erfolgen, dass erst ein Borstenkörper mit näherungsweise gleich langen Borsten hergestellt wird und dann eine Ablängung der Borsten wie erforderlich erfolgt. Dies ist derzeit bevorzugt. In jedem Fall bieten die Schnittkanten Vorteile bei der Aufnahme von Zellmaterial, weil sie, anders als etwa bei Trennung mittels Laserschnitt und dergleichen, über die Schnitt- bzw. Quetschkante besonders gut in Eingriff mit der Probeentnahmestelle treten können, um dort die zu sammelnden Zellen aufzunehmen. Gleichwohl wäre ein Laserschnitt oder ähnliches verwendbar.

Es ist möglich und vorteilhaft, wenn bei einem gynäkologischen Probeentnahmeinstrument weiter und/oder zusätzlich vorgesehen ist, dass die Kunststofffäden einen mittleren Durchmesser zwischen wenigstens 500 µm, bevorzugt wenigstens 600 µm und maximal 850 µm, bevorzugt maximal 800 µm aufweisen.

Diese Durchmesser sind gerade bei Polyamid- bzw. Aramid-Fasern bevorzugt, weil sie genügend Steifigkeit bieten, um einiges Zellmaterial mitzunehmen, wenn die Borsten an der Cervix bzw. der Scheidenwand entlang bewegt werden, andererseits aber nicht so unflexibel sind, dass Verletzungen zu befürchten sind. Es sei erwähnt, dass die Borsten typisch unmittelbar nach der Fertigung senkrecht zur Achse abstehen werden und für die bevorzugte Kelchform der äußeren Borsten, die insbesondere eine bessere Probenahme und eine bessere Sichtkontrolle erlaubt, aus dieser Radialorientierung entfernt werden müssen. Dies kann etwa durch eine Wärmbehandlung geschehen, aber typisch reicht es aus, wenn die Bürsten am Ort der Fertigung in ein enges Röhrchen eingebracht werden. Die typischen Zeiten bis zur Verwendung reichen dann, um die Kelchform zu gewährleisten.

Es ist möglich und vorteilhaft, wenn bei einem gynäkologischen Probeentnahmeinstrument weiter und/oder zusätzlich vorgesehen ist, dass wenigstens 750 Borstenenden auf maximal 3 cm Axialerstreckung vorhanden sind. Diese Liniendichte führt noch nicht zu einer wechselseitigen Behinderung der Punkte bei der Probeentnahme und stellt zugleich eine hinreichend hohe Zahl an Abgriffpunkten zur Verfügung. Gleichzeitig ist es bevorzugt, wenn eine nicht zu hohe Zahl an Abgriffpunkten verwendet wird, um Kapillareffekte und dergl. zu vermeiden. Es kann bevorzugt sein, nicht mehr als 1800 Abgriffpunkte auf 3cm Axialerstreckung zu verwenden, insbesondere bevorzugt unter 1500 Abgriffpunkte, weiter bevorzugt unter 1200 Abgriffspunkte und insbesondere weiter bevorzugt unter 1100 Abgriffpunkte. Dass bei kürzerer Axiallänge die Abgriffpunktezahl entsprechend zu verringern ist, sei vollständigkeitshalber erwähnt.

Es ist möglich und vorteilhaft, wenn bei einem gynäkologischen Probeentnahmeinstrument weiter und/oder zusätzlich vorgesehen ist, dass die Borsten mit der sie fixierenden Drahtanordnung als nach der Untersuchung abbrechbarer Endbereich an einem flexiblen Kunststoffstab angeordnet sind. Die Drahtanordnung, die typisch die Borsten durch Verdrillung zweier Drahtelemente fixiert, wird in einer bevorzugten Variante gemeinsam mit den Borsten an einem Kunststoffstab anzuordnen sein. Besonders bevorzugt ist es, wenn dabei der Endbereich, in welchem die die Borsten fixierende Drahtanordnung angeordnet ist, von einem flexiblen, für den Vorschub bis zum Entnahmeort bestimmten Kunststoffstab hinreichender Flexibilität abzubrechen ist. Ein Abbrechen gewährleistet ohne weiteres sofort, dass die Probeentnahmeinstrumente nur im Einwegverfahren benutzt werden können, weil sie nach einer gegebenen Untersuchung und dem daraufhin erfolgenden Abbrechen der Spitze durch manuelle Betätigung oder dergleichen gar nicht mehr neuerlich in die Scheide eingeführt werden könnten. Die Sollbruchstelle kann in den Kunststoffstab eingearbeitet sein, und zwar dergestalt, dass ein werkzeugfreies Abbrechen von Hand oder ein Abbrechen an einer Kante, insbesondere an der Kante eines für den Transport in ein Labor vorgesehenen Behälters möglich ist. Die Sollbruchstelle kann problemfrei so dimensioniert werden, dass ein Abbrechen innerhalb des Körpers nicht zu befürchten ist, aber gleichwohl ein manuelles Abbrechen leicht ermöglicht ist. Diesbezüglich sei darauf hingewiesen, dass typisch bei gynäkologischen Untersuchungen keine Gegenstände auf dem Weg zum Situs liegen, so dass keine großen mechanischen Belastungen am Stiel bzw. Schaft zu erwarten sind.

Es ist möglich und vorteilhaft, wenn bei einem gynäkologischen Probeentnahmeinstrument weiter und/oder zusätzlich vorgesehen ist, dass alle Borsten den gleichen Nominal-Durchmesser aufweisen. Dies ist vor allem fertigungstechnisch besonders vorteilhaft. Alternativ wäre aber auch möglich, Bündel von Borsten variierenden Durchmessers mittels des Drahtes zu fixieren oder entlang von dessen Verdrillungen unterschiedliche Dicken zu verwenden.

In einer Variante wird es sich bei dem gynäkologischen Probeentnahmeinstrument um eine Vaginalbürste handeln. Vaginalabstriche werden vor allem im Tumornachsorgescreening, aber auch präventiv vorgenommen. Die Vaginalbürste der vorliegenden Erfindung ist hierfür besonders geeignet. Insbesondere ist sie geeignet für cytologische Untersuchungen, HPV-Tests und molekularbiologische Tests. Zu diesen Zwecken wurden bislang die bei der Benutzung von den Patientinnen als eher unangenehm empfundenen Holzspatel oder abgerundete Kunststoffspatel verwendet. Mit der hier vorgeschlagenen Vaginalbürste mit einer Vielzahl von mehreren 100 Abgreifpunkten wird demgegenüber eine erhebliche Verbesserung der präanalytischen Sensitivität erreicht und zugleich auch an schwer zugänglichen Stellen ein Abstrich ermöglicht. Der vaginale Blindsack (nach Hysterektomie) ist nicht selten und vor allem in Situationen der Tumornachsorge nach Radiotherapie und ganz ausgeprägt nach sogenanntem Seit-zu-Seit-Kolpotomieverschluss durchaus nicht sphärisch, sondern häufig zipfelig asymmetrisch und ausgezogen, so dass "Retentionstaschen"' entstehen. Auch diese können aber mit den Borsten gut erreicht werden, was deshalb bedeutsam ist, weil gerade hier nicht selten ein sogenanntes Lokalrezidiv seinen Ausgang nimmt. Die Zellentnahme kann zudem schonend erfolgen, was insbesondere bei den typischerweise in diesem Kollektiv vorkommenden atrophen Verhältnissen besonders vorteilhaft ist.

Diese Vaginalbürste wird dann bevorzugt für die Einführung in die Vagina und die Probenentnahme aus der Vagina dimensioniert und angeordnet sein. Dafür kann die Vaginalbürste derart ausgebildet sein, dass die Borsten eine kranial abgerundete Bürstenkontur ergeben, bevorzugt mit einem kaudalen Durchmesser weg vom Zentrum von zwischen 20 mm und 29 mm, insbesondere bevorzugt zwischen 24 mm und 28 mm.

Erfindungsgemäß handelt es sich bei dem gynäkologischen Probeentnahmeinstrument um eine Cervixbürste, wobei bei dieser kaudal ein aufkelchender Borsten-Bereich vorhanden ist, dessen Borsten schräg zur Zentralachse abstehen und sich bis zu einem Durchmesser von maximal 28 mm, bevorzugt 26 mm, quer zur Achse erstrecken, wobei der aufkelchende Borstenbereich einen äußeren Kranz längerer Borsten und zumindest einen davon umfassten inneren Kranz von Borsten aufweist, wobei bevorzugt die Borsten des äußeren Borstenkranzes zumindest um so viel länger als die Borsten des inneren Borstenkranzes sind, dass während der Phase des Einführens in die Patientin sich wenigstens ein wesentlicher Teil der äußeren Borsten axial weiter nach vorne erstreckt als die Borsten des inneren Borstenkranzes. Was die Frage angeht, ab wann der Anteil der äußeren Borsten, die näher am Einführende des Probeentnahmeinstrumentes enden als die Borsten des inneren Borstenkranzes, als wesentlich angesehen werden kann, sei darauf hingewiesen, dass die Cervixbürste typisch unter Sicht durch ein Speculum hindurch eingeschoben wird und der vom Borstenkelch umgebende Borsten-Zentralbereich zur Sammlung von Zellen in den Gebärmutterhals eingeschoben werden soll. Dabei wird dann besonders gut sichtbar, dass die kelchbildenden Borsten weiter aufgespreizt werden und in Anlage an die äußeren Bereiche des Muttermundes kommen. Als wesentlich ist vor diesem Hintergrund der Anteil an sich weit nach vorne erstreckenden Borsten jedenfalls dann anzusehen, wenn klar erkennbar ist, wann dies der Fall ist. Die bevorzugte Länge der äußeren Borsten stellt dann einerseits sicher, dass quasi anatomieunabhängig bis hin zu hinreichend weit von der Gebärmutterhalsöffnung entfernt liegenden Stellen Probenmaterial gewonnen wird, während zugleich eine hervorragende Sichtkontrolle der Einschubtiefe durch die Borstenverformung gewährleistet ist. Es ist also keine taktile Erfassung der Borstenverformung erforderlich, sondern es kann statt dessen auch eine Verformung visuell beobachtet werden. Bei Herausbewegen der Bürste nach - unter Drehen erfolgter Probengewinnung - wird sich dann die Kelchform zurückbilden.

Die bevorzugte Kelchgestaltung gewährleistet bei einer Cervixbürste ein optimales Aufnehmen von Zellen, wobei die Sammelpunkte zu unterschiedlichen Zeitpunkten ansprechen können. So kann zunächst im Bereich der Außenborsten Kontakt aufgenommen werden und wenn die äußeren Kelchborsten in Kontakt getreten sind, werden auch die inneren Borsten des aufkelchenden Bereiches in Kontakt treten, typisch näher am Muttermund. Es wird durch die Bürstenform dabei nicht nur eine nominell hohe Zahl an Kontakten erreicht, sondern es wird auch gewährleistet, dass auf jeder der Borsten tatsächlich eine sinnvolle Menge an Zellmaterial aufgenommen werden kann und die Fäden sich nicht wechselseitig bei der Aufnahme behindern.

Die Cervixbürste wird bevorzugt kranial des aufkelchenden Borstenbereiches durch einen Kurzborstenbereich fortgesetzt. Bei der Cervixbürste ist es vorteilhaft, wenn kranial des aufkelchenden Borstenbereiches ein bis zum Bereich der Ektocervix einführbarer Kurzborstenbereich angeordnet ist, mit einem Durchmesser derart, dass der Durchmesser des äußeren Borstenkranzes im Kelchbereich wenigstens 4 mal, bevorzugt wenigstens 5 mal, insbesondere zwischen 5,5 mal und 7 mal größer ist als der Durchmesser des Kurzborstenbereiches, wobei die Kurzborsten allgemein radial von der Achse abstehen und die Kurzborsten eine Helixstruktur mit voneinander beabstandeten Windungen bilden. Der Begriff "allgemein radial" wird hier schon deshalb verwendet, weil herstellungsbedingt nicht gewährleistet werden kann, dass die Kurzborsten ganz exakt senkrecht von der Zentralachse abstehen. Es wird aber für den Fachmann ersichtlich sein, dass etwa bei "Eindrillen" der Kurzborsten zwischen zwei Drahtabschnitten während der Herstellung der Bürste allenfalls geringe Abweichungen auftreten werden.

In einer besonders bevorzugten Variante können im Bereich der aufkelchenden Borsten über 200 Fadenabgreifpunkte durch die Borsten gebildet sein, beispielsweise durch über 240 Borsten. Diese stellen Cervixabgreifpunkte dar. Die angegebenen Durchmesser sind im Übrigen größer als der in morphometrischen Studien ermittelte maximale Durchmesser von 22 mm einer Ektopie, vgl. die Studien von Przybora LA, Plutowa A. "Histological topography of carcinoma in situ of the cervix uteri." Cancer. 1959 Mar-Apr;12(2):263-77 bzw. Jacobson DL, Peralta L, Farmer M, Graham NM, Wright TC, Zenilman J. "Cervical ectopy and the transformation zone measured by computerized planimetry in adolescents." Int J Gynaecol Obstet. 1999 Jul;66(1):7-17.

Im Kelchbereich können dabei bevorzugt über 500 Fadenabgriffpunkte vorhanden sein, bevorzugt zwischen 500 und 700, insbesondere bevorzugt mehr als 550 und/oder weniger als 600.

Es ist mit der Cervixbürste, insbesondere in den bevorzugten Ausführungsformen, eine bestimmte, für den sehr weit überwiegenden Teil aller Patientinnen ausreichende Einführtiefe nicht nur erzielbar, sondern auch ohne weiteres reproduzierbar, was eine besonders einfache und standardisierte Probeentnahme erlaubt. Auf diese Weise wird zudem ungeachtet der kurzen Borsten ein unangenehmes Gefühl bei der Einführung der Borsten vermieden, zumindest aber verringert. Da die Cervixbürste bis zum Kontakt mit der Ektocervix eingeführt werden muss, wird zugleich eine zu hohe endozervikale Entnahme vermieden, wobei die dünnen Borstenhaare zu einer auch für die Patientin schonenden und überdies blutungsarmen Zellgewinnung führen, was sowohl für die Patientin wie auch für die nachfolgenden Laboruntersuchungen eindeutig vorteilhaft ist.

Bei dem gynäkologischen Probeentnahmeinstrument ist es weiter und/oder alternativ möglich, dass kranial am Ende des Drahtes ein Kunststoffkügelchen vorhanden ist, insbesondere aus einem flüssig auf das Drahtende aufgebrachten, auf dem Drahtende ausgehärteten Tropfen Kunststoffmaterial. Dieser bietet Schutz und ist zudem beim Eingehen in die eher engen, juvenilen oder altersatrophen Cervikalkanäle insbesondere bei der Cervixbürste hilfreich. Auf diese Weise wird ungeachtet der kurzen Borsten ein unangenehmes Gefühl bei der Einführung der Borsten vermieden, zumindest aber verringert. Es sei erwähnt, dass ein solcher Tropfen Kunstoffmaterial insbesondere für die Cervixbürste vorteilhaft ist.

Die Erfindung wird im Folgenden nur beispielhaft unter Bezugnahme auf die Zeichnung beschrieben. In dieser ist dargestellt durch
- Fig. 1: eine perspektivische Ansicht einer Cervixbürste nach der vorliegenden Erfindung;
- Fig. 2: die Cervixbürste von Fig. 1 in der Seitenansicht;
- Fig. 3: eine perspektivische Ansicht einer Vaginalbürste nach der vorliegenden Erfindung.

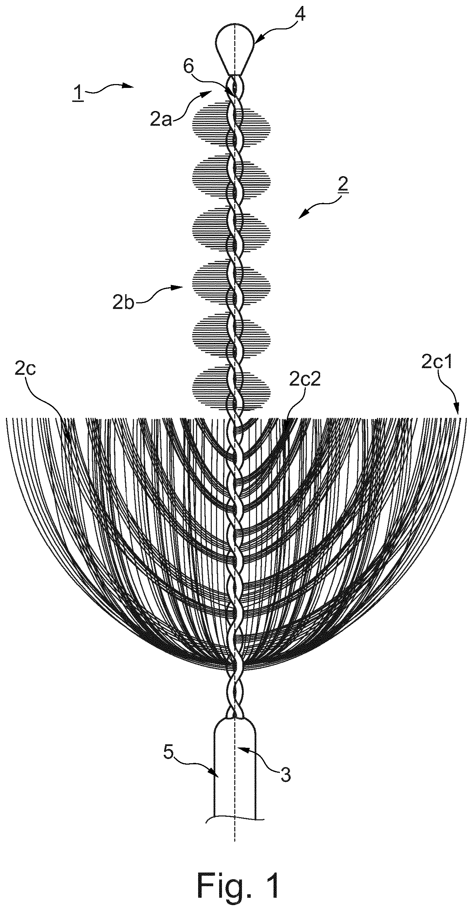

Nach Fig. 1 umfasst ein allgemein mit 1 bezeichnetes gynäkologisches Probeentnahmeinstrument 1 kranial eine Vielzahl von Borsten, die an einer sich in Axialrichtung erstreckenden Drahtanordnung fixiert sind, wobei die Borsten aus wenigstens 600 Kunststofffäden mit einem Durchmesser zwischen 350 µm und 900 µm und mit einer axialen Liniendichte von wenigstens 600 Fäden auf maximal 3 cm Axialerstreckung gebildet sind.

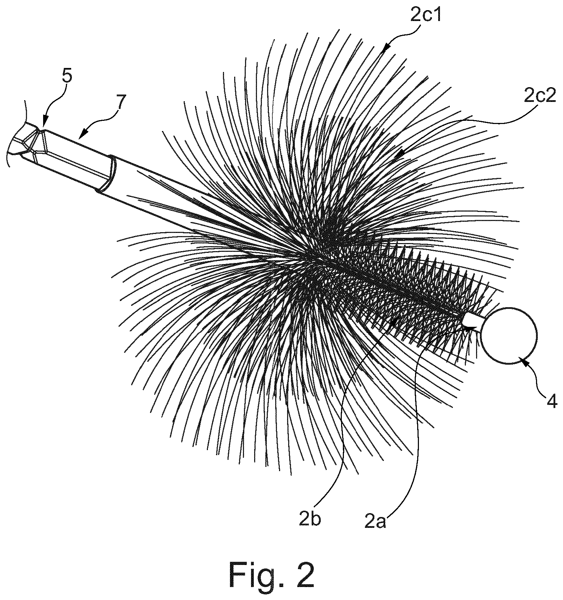

Das gynäkologische Probeentnahmeinstrument 1 ist in dem in Figuren 1 und 2 dargestellten Ausführungsbeispiel eine Cervixbürste zur Entnahme von Material aus dem Bereich des Muttermundes, insbesondere auch aus dem unmittelbaren Außenbereich um den Muttermund herum. Die dargestellte Cervixbürste ist in einem Röhrchen aufnehmbar, das so dimensioniert ist, dass die nach Produktion zunächst noch radial abstehenden längeren Borsten während des Transportes zum anwendenden Arzt bzw. während einer Lagerung die gewünschte Kelchform annehmen, wozu die zunächst noch radial abstehenden längeren Borsten im Röhrchen umgebogen werden.

Die Kunststofffäden sind in den dargestellten Ausführungsformen zwischen zwei entlang der Bürstenachse miteinander verdrillten Drahtstücken fixiert, wobei Borstenabschnitte zwischen die Drahtstücke gebracht und dann die Drähte kontinuierlich miteinander verdreht sind. Diese Fixierung erlaubt problemfrei, die gewünschten hohen Liniendichten durch Abstimmung der Verdrillungs-Festigkeit, Steigung entlang der Achse, Dichte der zwischen die Drähte gelangenden Borsten usw. zu erreichen und dabei auch die gewünschten Gesamtzahlen an Fäden bzw. Borsten einer erfindungsgemäßen Bürste zu erzielen.

Die Kunststofffäden sind im vorliegenden Fall aus Polyamid-Material (das unter dem Handelsnamen Nylon bekannt ist) hergestellt.

Die Borsten sind - im vorliegenden Fall nach der Fixierung in den verdrillten Drahtstücken - so abgelängt, dass sich für das dargestellte gynäkologische Probeentnahmeinstrument der vorliegenden Erfindung als Cervixbürste ein Bürstenaufbau ergibt, der drei unterschiedliche Bereiche erkennen lässt, nämlich - von kranial nach kaudal - ein borstenfreies proximales, d.h. dem Patientin-Inneren bei Benutzung zugewandtes Ende, ein Kurzborstenbereich für die Einführung in die Endocervix und ein langer Borstenbereich für die Probeentnahme aus dem Bereich der Ektocervix. Dies ist besonders gut in Fig. 2 zu erkennen. Am oberen borstenfreien Ende ist ein Tropfen Kunststoffmaterial über die miteinander verdrillten Drahtstücke aufgetropft, unter anderem, um die Patientin vor Verletzungen durch das sonst spitze Ende zu schützen. Es sei diesbezüglich erwähnt, dass die beiden Drahtstücke entlang ein und desselben durchgehenden einstückigen Drahtes gebildet sein können, der an der Spitze auf sich zurückgebogen ist.

Kaudal der Spitze ist zunächst ein Abschnitt mit hier etwa 240 kurzen Borsten und einer klaren Wendelstruktur vorgesehen. Die Borsten haben einen Durchmesser zwischen 600 µm und 800 µm und eine Länge weg vom axialen Zentrum um etwa 2 mm, d.h. sie bilden eine Wendel mit Durchmesser von ca. 4 mm. Die klar erkennbare Helixstruktur der Borsten, die zu axialen Zwischenräumen zwischen den Borstenenden führt, bedingt, dass einerseits leicht eine drehende Einführung in die Cervix vorgenommen werden kann, was eine feinfühligere Behandlung ermöglicht, und gewährleistet überdies ein Separiert-Bleiben von an den Borsten vorhandenem Zellmaterial zumindest dort, wo eine nur geringe Schleim-Mitnahme auftritt. Ermöglicht wird überdies eine Probeentnahme im Muttermundeintrittsbereich.

Näher am Griffteil des Schaftes, d.h. kaudal vom Kurzborstenbereich, ist ein Langborsten-Bereich vorgesehen. Die langen Borsten des Langborsten-Bereiches sind vom Schaft weg nach vorne aufgekelcht, ähnlich einer Tulpenblüte, die weg von ihrem Stängel aufkelcht, wobei sich die äußeren Borsten weiter nach kranial vorne erstrecken als die inneren Borsten. Das Aufkelchen kann erreicht werden, indem nach dem Eindrillen der Nylonfäden zwischen die miteinander zu verdrillenden Drahtstücke die auf die entsprechende Länge geschnittenen Borsten mit dem kaudalen (dem Arzt zugewandten) Ende der Drahtstücke in einen Kunststoffstab, an welchem sich das Instrument greifen und zum Situs einschieben lässt, eingegossen werden und dann der Stab mit dem Bürstenabschnitt in ein Transportrohr aufgenommen wird, das einen Durchmesser kleiner als der Durchmesser der inneren Borsten besitzt, und zwar unter Einschieben zunächst des kaudalen Endes in das Transportrohr, welches einen deutlich geringeren Durchmesser aufweist als die Länge der kaudalen Borsten. Indem die Anordnung mit dem Schaft voran in das Röhrchen geschoben wird, werden die längeren Nylonfäden nach kranial umgelegt, was allein durch die zwischen der Herstellung und dem Transport zum anwendenden Arzt typisch erforderliche Zeit zur gewünschten Aufkelchung führt.

Wie in Fig. 1 ersichtlich, sind die äußeren Kelchfäden länger als die inneren. In der bevorzugten Variante sind etwa 560 Fadenpunkte im Kelchbereich vorgesehen. Die Fäden ergeben im Ausführungsbeispiel einen Durchmesser von 24 mm im Innenkelchbereich und etwa 28 mm im äußeren Bereich. Diese Durchmesser sind einsichtiger Weise ohne weiteres zu variieren, z.B. um +-10 bis 15%, obgleich sich die angegebenen Durchmesser in der Praxis gut bewähren.

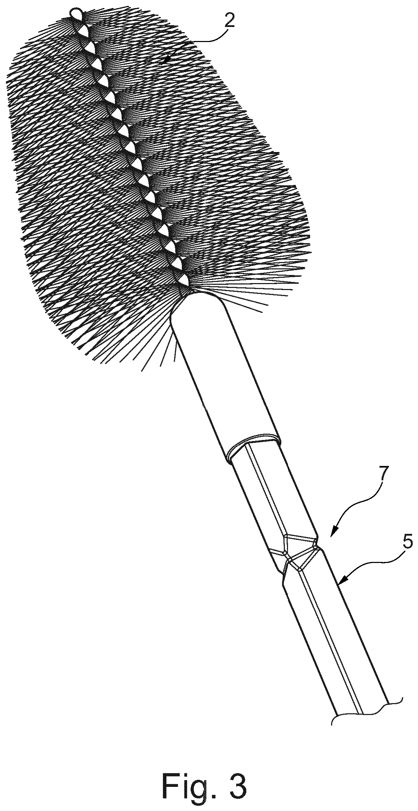

Die Schaft-Anordnung ist, wie am insoweit identischen zweiten Ausführungsbeispiel von Fig. 3 erkennbar, mit einer Sollbruchstelle dicht nahe dem Borstenbereich gebildet, wobei die Sollbruchstelle aber so weit vom Borstenbereich entfernt ist, dass zum Abbrechen des Borstenendes vom Griffteil des Schaftes noch ein ausreichender Bereich verbleibt, an dem das Borstenende ergriffen werden kann. Dieses am Borstenende bei Abtrennen verbleibende Teilstück reicht auch, um im Labor die für die Entfernung des gewonnenen Probenmaterials von der Bürste erforderliche Manipulation vorzunehmen. Der Schaft ist im Übrigen so dimensioniert, dass sein kaudales Ende bei Benutzung auch bei Patientinnen mit langer Scheide noch außerhalb der Scheide verbleibt. Nahe dem kaudalen Ende kann der Schaft insbesondere vollständig rund gebildet sein, um ein einseitiges oder Hin- und Herdrehen zwischen Daumen und Zeigefinger zu erleichtern.

In der Verwendung wird das gynäkologische Probeentnahme-Instrument unter Sicht durch ein Speculum hindurch bis an den Situs geführt. Das Erreichen des Situs, d.h. der richtigen Einschubtiefe, kann durch Beobachtung der Verformung der kelchbildenden Borsten leicht visuell erfasst werden. Dies ist bereits mit bloßem Auge gut möglich, kann aber auch koloskopisch erfolgen. Dann wird das gynäkologische Probeentnahme-Instrument am Situs drehend bewegt, bevor es wieder herausgezogen wird. Dabei gelangt zu entnehmendes Probenmaterial auf die Borsten. Die Flexibilität der Borsten bedingt dabei zugleich, dass Material aus jenen Schichten abgetragen wird, die für typische Untersuchungen besonders relevant sind. Dass es sich hierbei um sehr dünne Gewebeschichten handelt, sei erwähnt. Zugleich sei erwähnt, dass neben der Cervixkarzinomprophylaxe, bei welcher der HP-Virus kausal eine Schlüsselrolle besitzt, mit dem Abstrichinstrument auch Material für andere mikrobiologische Test gewonnen werden kann, wie zum Beispiel für Chlamydien, Trichomonaden, Gonokokken, Herpes simplex, A-Streptokokken, Treponema pallidum usw.. Erwähnt sei hier, dass aus den für Labor-Untersuchungen besonders relevanten Bereichen von Endo- und Ektozervix und dort aus der besonders bedeutsamen Schicht gleichzeitig mit nur einem einzigen Instrument Probenmaterial gesammelt werden kann, wobei überdies auch von ungeübtem Personal durch Beobachten einer einfach sichtbaren Verformung die Einhaltung der korrekten Eindringtiefe gewährleistet werden kann.

Dann kann die Bürste aus dem Körper der Patientin entfernt werden und die Zellen können auf einem Glasobjektträger ausgestrichen werden. Wenn für die nachgestellten Tests gewünscht, kann das Vorderende nahe der Bürste abgebrochen werden, und zwar indem es in einen Transportbehälter eingebracht wird und durch Abknicken im Behälter zurückbleibt. Nach ordnungsgemäßer Beschriftung usw., wird es in ein Labor transportiert, wo die entnommenen Proben untersucht werden. Geschieht dies durch Abstrich auf einen mikroskopischen Objektträger, ergibt sich durch die feine Verteilung der entnommenen Probe auf den vielen Fäden eine gut mikroskopierbare dünne Schicht. Muss hingegen das Probenmaterial in eine Lösung für weitere (cytologische oder andere) Untersuchungen überführt werden, ist dies ebenfalls ohne Probleme möglich.

Ein weiteres Ausführungsbeispiel ist in Fig. 3 gezeigt. Das dort dargestellte gynäkologische Probeentnahmeinstrument stellt eine Vaginalbürste dar, die eine für die vaginale Probeentnahme optimierte Bürstenform besitzt. Dazu hat die vaginale Bürste von Fig. 3 einen allgemein konusförmigen Borstenbereich, der kranial vergleichsweise stumpf zuläuft, d.h. die proximalen Borsten bilden immer noch einen Durchmesser von über 5 mm, im dargestellten Ausführungsbeispiel von sogar 10 mm.

Dies macht evtl. auch das Aufbringen eines Kunststoff-Tropfens auf das kaudale Ende entbehrlich. Insbesondere, wenn die die Borsten einklemmenden fixierenden Drahtelemente einstückig aus einem durchgehenden Stück Draht gebildet sind, das am proximalen Ende mit hinreichend großem Krümmungsradius auf sich zurückgebogen ist, wird bereits - gerade in Kombination mit den noch hinreichend langen proximalen Borsten - eine Verletzung der Scheidenwand vermieden. Gleichwohl wird typisch das Vorhandensein eines Kunststofftropfens auch bei der Vaginalbürste bevorzugt, weil selbst bei Produktionsfehlern und dergleichen das Verletzungsrisiko verringert ist. Es sei betont, dass es auch bei der Cervixbürste möglich und bevorzugt ist, die die Borsten einklemmenden fixierenden Drahtelemente einstückig aus einem durchgehenden Stück Draht zu bilden, welcher am proximalen Ende mit hinreichend großem Krümmungsradius auf sich zurückgebogen ist. Dort ist aber das Vorhandensein des zusätzlichen Kunststoff-Tropfens wegen der geringen Borstendurchmesser des proximalen Helixbereiches noch klarer bevorzugt als bei der Vaginalbürste, die zumindest im Ausführungsbeispiel ohne Kunstofftropfen dargestellt ist.

Die langen, kranial vorgesehenen Borsten der Vaginalbürste verhindern, und zwar selbst bei einem ungeschickten Arzt, dass die Scheidenwand bei Einführung verletzt wird.

Über die Gesamtausdehnung des Borstenbereiches, der sich etwa 20 mm axial erstreckt, sind im dargestellten Ausführungsbeispiel 800 Faden-Angriffspunkte vorgesehen, die sich etwa gleichmäßig über den Umfang verteilen. Damit wird eine gute Probeentnahme ähnlich wie bei der Cervixbürste erreicht, genauso wie eine gute Abgabe des entnommenen Probematerials für die Untersuchung im Labor in Lösung oder auf einen mikroskopischen Träger.

Es sei erwähnt, dass die Fertigung ohne weiteres preiswert erfolgen kann und dass im Übrigen Borsten aus anderem Material außer Nylon einsetzbar wären.

### Bezugszeichenliste

- 1: = Gynäkologisches Probeentnahmeinstrument;
- 2: = Borsten;
- 2a: = borstenfreies proximales, d.h. dem Patientin-Inneren bei Benutzung zugewandtes Ende;
- 2b: = Kurzborstenbereich für die Einführung in die Endozervix;
- 2c: = aufkelchender Borstenbereich für die Probeentnahme aus dem Bereich der Ektozervix;
- 2c1: = äußeren Kelchfäden (Fig. 2) ;
- 2c2: = innere Kelchfäden (Fig. 2) ;
- 3: = Bürstenachse;
- 4: = Tropfen;
- 5: = Stiel;
- 6: = Drahtstücke;
- 7: = Sollbruchstelle (Fig. 3).

## Patentansprüche

1. Gynäkologische Cervixbürste (1),
die zum Patientenende hin eine Vielzahl von Borsten aufweist, die an einer sich in Axialrichtung erstreckenden Drahtanordnung fixiert sind,
wobei die Borsten (2) aus wenigstens 600 Kunststofffäden mit einem Durchmesser zwischen 350 µm und 900µm, einer axialen Liniendichte von wenigstens 600 Fäden auf maximal 3 cm Axialerstreckung gebildet sind, wobei weiter patientenaußenseitig ein aufkelchender Borsten-Bereich vorhanden ist, dessen Borsten (2) schräg zur Zentralachse abstehen und
sich bis zu einem Durchmesser von 28 mm, bevorzugt 26 mm quer zur Achse erstrecken, und der aufkelchende Borstenbereich (2c) einen äußeren Kranz (2c1) längerer Borsten (2) und zumindest einen davon umfassten inneren Kranz Borsten (2c2) aufweist,
wobei die Borsten des äußeren Borstenkranzes zumindest um so viel länger als die Borsten des inneren Borstenkranzes sind,
dass sich wenigstens ein wesentlicher Teil der äußeren Borsten weiter nach vorne erstreckt als die Borsten des inneren Borstenkranzes.

2. Gynäkologische Cervixbürste (1) nach dem vorhergehenden Anspruch, **dadurch gekennzeichnet, dass** die Kunststofffäden aus Polyamid hergestellt sind.

3. Gynäkologische Cervixbürste (1) nach einem der vorhergehenden Ansprüche, **dadurch gekennzeichnet, dass** die Kunststofffäden mechanisch abgeschnitten bzw. abgeschert sind.

4. Gynäkologische Cervixbürste (1) nach einem der vorhergehenden Ansprüche, **dadurch gekennzeichnet, dass** die Kunststofffäden einen mittleren Durchmesser zwischen wenigstens 500 µm, bevorzugt wenigstens 600 µm und maximal 850 µm, bevorzugt maximal 800 µm aufweisen.

5. Gynäkologische Cervixbürste (1) nach einem der vorhergehenden Ansprüche, **dadurch gekennzeichnet, dass** wenigstens 750 Borstenenden auf maximal 3 cm Axialerstreckung vorhanden sind.

6. Gynäkologische Cervixbürste (1) nach einem der vorhergehenden Ansprüche, **dadurch gekennzeichnet, dass** die Borsten (2) mit der sie fixierenden Drahtanordnung als nach der Untersuchung abbrechbarer Endbereich an einem flexiblen Kunststoffstab angeordnet sind.

7. Gynäkologische Cervixbürste (1) nach einem der vorhergehenden Ansprüche, **dadurch gekennzeichnet, dass** alle Borsten (2) den gleichen Nominal-Durchmesser aufweisen.

8. Gynäkologische Cervixbürste (1) nach dem vorhergehenden Anspruch, **dadurch gekennzeichnet, dass**
kranial des aufkelchenden Borstenbereiches ein bis zum Bereich der Ektocervix einführbarer Kurzborstenbereich angeordnet ist,
wobei der Durchmesser des äußeren Borstenkranzes im Kelchbereich wenigstens 4 mal, bevorzugt wenigstens 5 mal, insbesondere zwischen 5,5 mal und 7 mal größer ist als der Durchmesser des Kurzborstenbereiches,
die Kurzborsten allgemein radial von der Achse abstehen und
die Kurzborsten eine Helixstruktur mit voneinander beabstandeten Windungen bilden
und/oder wobei
am proximalen Ende das Drahtende mit einem Kunststoffschutz versehen ist, insbesondere aus einem flüssig auf das Drahtende aufgebrachten, auf dem Drahtende ausgehärteten Tropfen Kunststoffmaterials.

## Claims

1. Gynaecological cervical brush (1),
which has a multiplicity of bristles towards the patient end,
which bristles are fixed to a wire assembly that extends in an axial direction,
wherein
the bristles (2) are formed of
at least 600 plastic filaments
with a diameter of between 350 µm and 900 µm
and with an axial line density of at least 600 filaments over a maximum axial extent of 3 cm, wherein further to the outside of the patient, a cup-shaped bristle region is present
whose bristles (2)
protrude obliquely with respect to the central axis
and
extend up to a diameter of 28 mm, preferably 26 mm, transversely with respect to the axis,
and
the cup-shaped bristle region (2c) has
an outer ring (2c1) of longer bristles (2)
and,
enclosed by the latter, at least one inner ring of bristles (2c2),
wherein the bristles of the outer bristle ring are longer than the bristles of the inner bristle ring by at least such an extent
that at least a substantial part of the outer bristles extends further forwards than the bristles of the inner bristle ring.

2. Gynaecological cervical brush (1) according to the preceding claim, **characterized in that** the plastic filaments are produced from polyamide.

3. Gynaecological cervical brush (1) according to either of the preceding claims, **characterized in that** the plastic filaments are cut off or sheared off mechanically.

4. Gynaecological cervical brush (1) according to any one of the preceding claims, **characterized in that** the plastic filaments have an average diameter of between at least 500 µm, preferably at least 600 µm, and at most 850 µm, preferably at most 800 µm.

5. Gynaecological cervical brush (1) according to any one of the preceding claims, **characterized in that** at least 750 bristle ends are present over a maximum axial extent of 3 cm.

6. Gynaecological cervical brush (1) according to any one of the preceding claims, **characterized in that** the bristles (2), with the wire assembly fixing them, are arranged as an end region, that can be broken off after the examination, on a flexible plastic rod.

7. Gynaecological cervical brush (1) according to any one of the preceding claims, **characterized in that** all of the bristles (2) have the same nominal diameter.

8. Gynaecological cervical brush (1) according to the preceding claim, **characterized in that**
a short-bristle region insertable as far as the region of the ectocervix is arranged cranially with respect to the cup-shaped bristle region,
wherein the diameter of the outer bristle ring in the cup region is at least 4 times, preferably at least 5 times, in particular between 5.5 times and 7 times greater than the diameter of the short-bristle region,
the short bristles generally protrude radially from the axis
and
the short bristles form a helix structure with windings spaced apart from one another,
and/or wherein
at the proximal end, the wire end is provided with a plastic protector, in particular made of a plastic material applied in the form of a liquid drop to the wire end and cured on the wire end.

## Revendications

1. Brosse gynécologique pour col de l'utérus (1),
qui présente une pluralité de poils vers l'extrémité côté patient,
qui sont fixés à un agencement de fil métallique s'étendant dans la direction axiale,
où
les poils (2) sont formés par
au moins 600 fils en matière plastique
d'un diamètre compris entre 350 µm et 900 µm,
d'une densité linéaire axiale d'au moins 600 fils sur une extension axiale maximale de 3 cm, une zone de poils en forme de coupe étant présente plus près de l'extérieur du patient,
dont les poils (2)
font saillie obliquement de l'axe central
et
s'étendent transversalement à l'axe jusqu'à un diamètre de 28 mm, de préférence 26 mm,
et
la zone de poils en forme de coupe (2c) présente
une couronne extérieure (2c1) de poils plus longs (2) et
au moins une couronne intérieure de poils (2c2) entourée par celle-ci,
les poils de la couronne de poils extérieure étant au moins aussi longs que les poils de la couronne de poils intérieure,
au moins une partie importante des poils extérieurs s'étend plus vers l'avant que les poils de la couronne de poils intérieure.

2. Brosse gynécologique pour col de l'utérus (1) selon la revendication précédente, **caractérisée en ce que** les fils en matière plastique sont fabriqués en polyamide.

3. Brosse gynécologique pour col de l'utérus (1) selon l'une quelconque des revendications précédentes, **caractérisée en ce que** les fils en matière plastique sont coupés ou cisaillés mécaniquement.

4. Brosse gynécologique pour col de l'utérus (1) selon l'une quelconque des revendications précédentes, **caractérisée en ce que** les fils en matière plastique présentent un diamètre moyen compris entre au moins 500 µm, de préférence au moins 600 µm, et au maximum 850 µm, de préférence au maximum 800 µm.

5. Brosse gynécologique pour col de l'utérus (1) selon l'une quelconque des revendications précédentes, **caractérisée en ce qu'**au moins 750 extrémités de poils sont présentes sur une extension axiale maximale de 3 cm.

6. Brosse gynécologique pour col de l'utérus (1) selon l'une quelconque des revendications précédentes, **caractérisée en ce que** les poils (2) avec l'agencement de fil métallique qui les fixe sont agencés sur une tige en matière plastique flexible sous forme de zone d'extrémité pouvant être cassée après l'examen.

7. Brosse gynécologique pour col de l'utérus (1) selon l'une quelconque des revendications précédentes, **caractérisée en ce que** tous les poils (2) présentent le même diamètre nominal.

8. Brosse gynécologique pour col de l'utérus (1) selon la revendication précédente, **caractérisée en ce que** une zone à poils courts pouvant être introduite jusqu'à la zone de l'exocol est agencée au-dessus de la zone de poils en forme de coupe,
le diamètre de la couronne de poils extérieure dans la zone de la coupe étant au moins 4 fois, de préférence au moins 5 fois, notamment entre 5,5 fois et 7 fois supérieur au diamètre de la zone à poils courts,
les poils courts faisant saillie généralement radialement de l'axe
et
les poils courts formant une structure hélicoïdale avec des spires espacées les unes des autres
et/ou
à l'extrémité proximale, l'extrémité de fil métallique étant pourvue d'une protection en matière plastique, notamment constituée d'une goutte de matière plastique appliquée à l'état liquide sur l'extrémité de fil métallique, durcie sur l'extrémité de fil métallique.
